(19) Europäisches Patentamt
European Patent Office
Office européen des brevets

(11) EP 1 214 878 A1

# (12) EUROPEAN PATENT APPLICATION

(43) Date of publication:
**19.06.2002 Bulletin 2002/25**

(51) Int Cl.[7]: **A01K 1/015**, A61L 9/01, A61L 15/46

(21) Application number: **00870301.9**

(22) Date of filing: **15.12.2000**

(84) Designated Contracting States:
**AT BE CH CY DE DK ES FI FR GB GR IE IT LI LU MC NL PT SE TR**
Designated Extension States:
**AL LT LV MK RO SI**

(71) Applicant: **The Procter & Gamble Company
Cincinnati, Ohio 45202 (US)**

(72) Inventors:
- **Stoddart, Barry
Low Fell, NE9 5AP Gateshead (GB)**
- **Narinx, Emmanuel Pierre Jacques
4053 Embourg (BE)**

(74) Representative: **Canonici, Jean-Jacques et al
BVBA Procter & Gamble Europe SPRL,
Temselaan 100
1853 Strombeek-Bever (BE)**

## (54) Methods, compositions and articles for control of malodor produced by urea-containing body fluids

(57) Disclosed are methods, compositions and articles suitable for controlling the undesirable ammonia odor produced by excreted or secreted body fluids, e. g., urine and/or sweat, and residues thereof. Such methods, compositions and articles utilize certain urease inhibitor complexes formed from a divalent metal ion and a polyanionic, preferably amine-based, chelating agent to prevent or minimize the urease-promoted degradation of urea (found in the body fluids) to malodorous ammonia.

EP 1 214 878 A1

## Description

### TECHNICAL FIELD/FIELD OF THE INVENTION

This invention relates to methods, compositions and articles suitable for controlling the undesirable ammonia odor produced as a consequence of the presence of excreted and secreted body fluids such as urine and/or sweat. The invention can take the form of a wide variety of products which can be used to practice the odor control method. Such products include cleaning compositions; odor control compositions; pet litter products; treated animal waste-based fertilizer, and absorbent articles such as diapers, other incontinence devices and pads, feminine protection products, and a wide variety of additional products which encounter body fluids.

### BACKGROUND OF THE INVENTION

It is, of course, well-known that secretion or excretion of body fluids from humans or other mammals can cause undesirable odor problems to arise in a number of situations. When, for example, urine is excreted into absorbent articles, such as diapers or incontinence pads, onto hard surfaces, such as those comprising or surrounding toilets or urinals, or into absorbent materials such as pet litter, odor problems can arise within a relatively short period of time.

The mechanism for odor production in these kinds of situations is fairly straightforward. Fresh urine, in fact, does not smell. However, aged urine malodor results when the urea in urine is degraded by urease enzyme which may also be present in the urine via contamination or present in environments into which the urine has been introduced. Breakdown of urea by urease enzyme results in the production of ammonia and carbon dioxide. It is the perception of the ammonia smell which people associate with urine malodor. A similar mechanism can cause odor problems with respect to other urea-containing body fluids such as sweat.

Prevention of urea degradation by urease enzymatic activity is neither simple nor straightforward. Many known inhibitors of enzymatic activity (e.g., urease inhibitors) are toxic or are unstable or are not sufficiently efficient or effective so as to be useful in the context of consumer or even industrial products for malodor control. While products suitable for management of body exudates are common, there is nevertheless a continuing need for the identification and development of additional products and methods which can be used to control the malodor problem associated with the excretion or secretion of mammalian body fluids such as urine or sweat.

### SUMMARY OF THE INVENTION

The present invention relates to products and methods which are suitable for preventing or minimizing ammonia odor produced by the degradation of urea in secreted or excreted body fluids. Such products and methods utilize, as a urease inhibitor, a selected type of complex formed from a polyanionic, and preferably amine-based, chelating agent and a divalent heavy metal ion. In such complexes, the metal ion is complexed at from 4 to 6 coordination sites, with one additional coordination site remaining available for binding with urease. Such chelated metal complexes must have a stability constant, K, such that $\log(10)K > 12.5$. An especially preferred complex of this type is the pentadentate chelant which comprises the copper salt of N-hydroxyethyl-ethylenediamine-triacetic acid (i.e., CuHEDTA).

The chelated metal complexes used in this invention are brought into contact with urea-containing body fluids, or the residues of such fluids, in order to prevent or minimize odor production caused by the presence and aging of such fluids or residues. Such contact can be brought about by incorporating the urease inhibitor complex, along with a delivery agent, delivery means or carrier, into a wide variety of products such as cleaning or treating compositions for application to bathroom fixtures such as toilets and urinals and to the floors and walls in proximity to such fixtures; laundry products; pet litter products; fertilizer compositions; absorbent articles such as diapers, incontinence pads, catamenial products, mattress pads and sweatbands; non-absorbent articles such as underwear, socks, bed clothing including sheets and mattress and pillow cases and covers; and the like.

### DETAILED DESCRIPTION OF THE INVENTION

The present invention relates to the use of urease inhibitors of a certain type to prevent, or at least minimize, odor produced by degradation of urea in secreted or excreted body fluids and/or residues of such body fluids. To form the compositions or articles of the present invention which can be used to carry out the odor reduction method herein, the urease inhibitor is combined with one or more additional elements suitable for delivering the urease inhibitor complex to the situs where urease inhibiting activity is needed. The urease inhibitor and the other "delivery" elements of the present invention are described in detail as follows:

Urease Inhibitor

The urease inhibitors useful herein are chelated metal complexes formed by reacting a divalent metal ion with a polyanionic chelating agent. The useful metals and chelating agents and the preparation of the chelated metal complexes therefrom as well as the requisite properties and characteristics of these complexes are set forth as follows:

Suitable metal ions for forming the urease inhibitor complexes used in this invention are those of divalent heavy metals. Such metals in ionic form include copper ($Cu^{2+}$), zinc ($Zn^{2+}$), nickel ($Ni^{2+}$), cobalt ($Co^{2+}$) and iron ($Fe^{2+}$). The divalent copper ion, $Cu^{2+}$, is the most preferred metal for forming the urease inhibitor complexes used herein.

These heavy metal ions are reacted with a chelating agent to form metal coordination complexes which function as urease inhibitors. The chelating agent can be any polyanionic moiety-containing organic material which will form 4, 5 or 6 coordinates with the divalent metal ion. Most preferred are those chelating agents which form a 5-coordinate complex with divalent metal ion. Upon chelation, there should be at least one additional coordination site remaining available within the complex for binding with urease. Preferably the chelating agent will form an octahedral complex with the divalent metal ion.

Any polyanionic chelating agent which will chelate the hereinbefore-described selected metals in a manner which provides the right number of coordination sites and the right stability constant characteristics may be employed to form the urease inhibitor complexes used herein. Thus polycarboxylates, such as substituted or unsubstituted carboxymethylcellulose materials, may be employed as chelating agents, as well as polycarboxylate compounds containing nitrogen, sulfur or phosphorus atoms. Nitrogen-containing materials, e.g. amine-based chelating agents, are preferred.

Amine-based chelating agents useful for forming the preferred urease inhibitor complexes used herein can include monoamine or polyamine species. Suitable monoamine-based chelating agents include nitrilotriacetic acid (NTA) of the formula: $N(CH_2COOH)_3$. Another suitable monoamine chelating agent is iminodisuccinic acid (IDS)of the formula: $HOOC-CH_2CH(COOH)-NH-CH(COOH)-CH_2-COOH$.

Suitable polyamines include carboxymethylated substituted ethylenediamine materials of the general formula:

$$R(CH_2COOH)N-(CH_2)_2-N-(CH_2-COOH)_2$$

wherein R is an organic moiety which does not form a coordination link with the heavy metal ion to be chelated therewith. Thus, for example, R can be $-(CH_2)_nCH_3$ or

- $(CH_2)_nOH$ wherein n can range from 0 to 8 or
- $(CH_2)_n-O-Si-(OCH_3)_3$, wherein n can range from 3 to 8.

An especially preferred diamine-based chelating agent is one of the above formula wherein R= $-(CH_2)_2OH$, i.e., N-hydroxyethyl-ethylenediamine triacetic acid or "HEDTA". Other possible chelating agents include amino acid derivatives such as those based on lysine. Chelated metal complexes formed from this type of chelants are disclosed, for example, in European Patent Publication EP-A-972,566; Published January 19, 2000.

The complexes formed from divalent metal ions and amine-based chelants must have certain stability characteristics in order to function effectively as odor-controlling urease inhibitors in the context of the present invention. Such chelated metal complexes are generally in equilibrium with their constituent simpler ions and molecules in accordance with the relationship, $AB \Leftrightarrow A + B$. The equilibrium or stability constant K for the complex, which is a measure of the stability of the complex, is defined in conventional fashion as:

$$K = \frac{[AB]}{[A]\,[B]}$$

wherein [AB], [A] and [B] are the molar concentrations of the several species in aqueous solution at reaction equilibrium (25 °C). The urease inhibitor complexes used in the present invention are those which have a stability constant K such that $\log(10)K > 12.5$. Preferred urease inhibitor complexes herein are those wherein $\log(10)K > 15$.

The chelated metal complexes as hereinbefore described can be prepared in conventional fashion by carrying out the chelation reaction in aqueous solution. Thus a source of metal ions, i.e., a water-soluble salt of the desired metal, can be added to an aqueous reaction mixture along with the amine-based chelating agent which will generally be in its free acid form. Reactant concentrations may be limited by their solubility in the reaction mixture. However, typically concentrations ranging from 0.01M to 1.0M may be employed, along with reaction temperatures which range from 1 °C to 50 °C and reaction times of from 5 seconds to several minutes.

Of all the chelated metal complexes useful as urease inhibitors herein, the most preferred is the pentadentate

chelant which is the copper salt of N-hydroxyethyl-ethylenediamine triacetic acid, i.e., Cu-HEDTA. Other preferred complexes are the copper salt of iminodisuccinic acid, i.e., Cu-IDS and the copper salt of nitrilotriacetic acid, e.g., Cu-NTA.

## Odor Control Methods

In its method aspects, the urease inhibitor complexes as hereinbefore described are brought into contact with urea-containing body fluids or residues of such fluids. Urea-containing body fluids include urine and sweat (perspiration). Such aqueous body fluids are those secreted or excreted by humans and by other mammals such as household pets. These fluids, once contaminated, or the environments in which they are found will generally contain microorganisms such as bacteria which produce the enzyme urease. Urease, in turn, will enzymatically degrade the urea present in the body fluids or residues thereof to form ammonia and carbon dioxide. Ammonia formation can then cause odor and can also elevate pH, for example in the region of skin, which in turn can promote other types of undesired enzymatic activity and can cause skin irritation.

In the methods of the present invention, the selected urease inhibitor complexes described above, having the right balance of urease binding activity and complex stability, when brought into contact with urea-containing body fluids or residues can serve to prevent or inhibit degradation of urea by urease. This in turn can prevent or minimize formation of odor-causing ammonia and can prevent or minimize the raising of ambient pH.

Depending upon the context of odor control and of pH control desired, contact of the urease inhibitor complexes herein with body fluids or their residues can be brought about by a variety of different means or procedures. For example, in one aspect of the method herein, such contact is brought about by contacting substrates which have been insulted with body fluids or residues thereof with a composition containing the urease inhibitor complex. In another aspect, such contact may be brought about by bringing body fluids or residues thereof into contact with an article which has the urease inhibitor complex associated therewith. In the composition and article aspects of this invention as described hereinafter, the various means and procedures for contacting the inhibitor complex with fluids or residues are described in greater detail.

In general, with respect to odor-controlling compositions, the urease inhibitor complex is brought into contact with the body fluids or residues by a delivery agent or carrier. In its simplest form, the delivery agent/carrier can comprise water but may also comprise a variety of other composition components as well.

In general, with respect to odor-controlling articles of manufacture or devices, the urease inhibitor complex is brought into contact with body fluids or residues by delivery means which may comprise one or more physical elements of the articles or devices with which the urease inhibitor complex is associated. Thus, for example, the urease inhibitor may be absorbed into, adsorbed on or chemically bonded to a substrate which forms part of the desired article.

## Malodor-Controlling Compositions

The urease inhibitor complex of the present invention can be utilized in a wide variety of odor-controlling compositions suitable for a wide variety of purposes. In its simplest form, odor control compositions herein can merely comprise combinations of the selected urease inhibitor complexes herein, or residues thereof, with a liquid or solid (e.g., granular) carrier. Thus, for example, very simple compositions can comprise aqueous solutions of the hereinbefore-described urease inhibitor complex or its precursor constituent components. Such aqueous compositions can be then used to treat objects, areas, substrates or environments which have been or are likely to be insulted or contacted with urea-containing body fluids or residues. Examples of utilization of such simple aqueous solutions of this type would be for treatment of hard surfaces of, in, around, or otherwise proximate to bathroom fixtures such as toilets, urinals, bidets, sinks, partitions, countertops and the like.

Compositions in the form of aqueous solutions can contain from 0.1% to 10% by weight of the urease inhibitor complex, more preferably from 0.5% to 5% by weight of the urease inhibitor complex. When the complex is to be formed *in situ* in aqueous solutions, the source of the heavy metal ions can generally be added in an amount which comprises from 0.25% to 7.5% by weight of the composition to be formed and chelating agent can be added in an amount which comprises from 1% to 10% by weight of the composition to be formed. The molar ratio of heavy metal source to polyanionic chelating agent can range from 0.75:1 to 1:0.75.

Compositions containing the selected urease inhibitor complex herein can also be provided in the form of cleaning compositions for a variety of substrates such as fabrics, carpets and hard surfaces. Such cleaning compositions may be aqueous or non-aqueous in nature and may be in dry (granular) or liquid form. Generally, odor-controlling cleaning compositions, in addition to the requisite urease inhibitor complex, will contain one or more detersive surfactants and/or detergent builder components.

Detersive surfactants can comprise from 0.1% to 50% by weight of the cleaning compositions herein. Preferably such compositions will comprise from 0.5% to 5% by weight of detersive surfactant. Detersive surfactants utilized

can be of the anionic, nonionic, zwitterionic, ampholytic or cationic type or can comprise compatible mixtures of these types. Detergent surfactants useful herein are described in U.S. Patent 3,664,961, Norris, Issued May 23, 1972; U.S. Patent 3,919,678, Laughlin et al., Issued December 30, 1975; U.S. Patent 4,222,905, Cockrell, Issued September 16, 1980; and in U.S. Patent 4,239,659, Murphy, Issued December 16, 1980. All of these patents are incorporated herein by reference. Of all the surfactants, anionics and nonionics are preferred.

Preferred anionic surfactants which are suitable for use in the odor-controlling cleaning compositions herein include the water-soluble salts, preferably the alkali metal, and ammonium salts, of organic sulfuric reaction products having in their molecular structure an alkyl group containing from about 10 to about 20 carbon atoms and a sulfonic acid or sulfuric acid ester group. (Included in the term "alkyl" is the alkyl portion of acyl groups.) Examples of this group of synthetic surfactants are a) the sodium, potassium and ammonium alkyl sulfates, especially those obtained by sulfating the higher alcohols ($C_8$-$C_{18}$ carbon atoms) such as those produced by reducing the glycerides of tallow or coconut oil; b) the sodium, potassium and ammonium alkyl polyethoxylate sulfates, particularly those in which the alkyl group contains from 10 to 22, preferably from 12 to 18 carbon atoms, and wherein the polyethoxylate chain contains from 1 to 15, preferably 1 to 6 ethoxylate moieties; and c) the sodium and potassium alkylbenzene sulfonates in which the alkyl group contains from about 9 to about 15 carbon atoms, in straight chain or branched chain configuration, e.g., those of the type described in U.S. Patents 2,220,099 and 2,477,383. Especially valuable are linear straight chain alkylbenzene sulfonates in which the average number of carbon atoms in the alkyl group is from about 11 to 13, abbreviated as $C_{11-13}$ LAS.

Nonionic surfactants for use, preferably in combination with the foregoing anionics, in the cleaning compositions herein are those of the formula $R^1(OC_2H_4)_nOH$, wherein $R^1$ is a $C_{10}$-$C_{16}$ alkyl group or a $C_8$-$C_{12}$ alkylphenol group, and n is from 3 to about 80. Particularly preferred are condensation products of $C_{12}$-$C_{15}$ alcohols with from about 5 to about 20 moles of ethylene oxide per mole of alcohol, e.g., $C_{12}$-$C_{13}$ alcohol condensed with about 6.5 moles of ethylene oxide per mole of alcohol.

The malodor-controlling cleaning compositions herein may also comprise from 0.1% to 80% by weight of a detergent builder. Preferably such compositions, especially when in liquid form, can comprise from 1% to 10% by weight of the builder component. Preferably such compositions, when in granular form, can comprise from 1% to 50% by weight of the builder component. Detergent builders are well known in the art and can comprise, for example, phosphate salts as well as various organic and inorganic nonphosphorus builders.

Water-soluble, nonphosphorus organic builders useful herein include the various alkali metal, ammonium and substituted ammonium polyacetates, carboxylates, polycarboxylates and polyhydroxy sulfonates. Examples of suitable nonphosphorus, inorganic builders include the silicates, aluminosilicates, borates and carbonates. Particularly preferred are sodium and potassium carbonate, bicarbonate, sesquicarbonate, tetraborate decahydrate, and silicates having a weight ratio of $SiO_2$ to alkali metal oxide of from 0.5 to 4.0.

Odor-controlling compositions, as hereinbefore described, in the form of treating compositions or cleaning compositions for fabrics, carpets and/or hard surfaces may employ urease inhibitor complexes which are in a form that renders them substantive to materials being contacted with such compositions. In particular, such compositions may employ urease inhibitors which are of the chelated metal type as described herein but which have been further chemically modified to render them capable of forming chemical bonds with the substrates they have been contacted with.

One type of composition which provides urease inhibitor complexes that are substantive to the materials being treated therewith comprises compositions wherein the urease inhibitor complex has been chemically modified to render it reactive with siliceous hard surfaces such as porcelain. For example, the chelating agent HETDA can be reacted with Cl- $(CH_2)_3$-O-Si-$(OCH_3)_3$ to form $R(CH_2COOH)N$-$(CH_2)_2$-N-$(CH_2$-$COOH)_2$ wherein R is -$(CH_2)_3$-O-Si-$(OCH_3)_3$. Such a material can form chemical bonds with porcelain surfaces when it is brought into contact for such surfaces via, for example, cleaning solutions, treating solutions, cleaning wipes or continuous introduction into toilet flush water. Upon metal chelation by reactive materials of this type, urease inhibitor complexes substantive to the treated hard surfaces are formed.

Another example of compositions which can be used to impart anti-malodor properties to substrates treated therewith are those which contain the urease inhibitor complex in a form which is readily reactive with the treated substrates under normal washing or treating conditions. In such compositions, the urease inhibitor complex can be reacted with materials which are conventionally used to create reactive dyes for use on fabrics or carpets. For example, heterocyclic nitrogen-containing compounds having appropriately selected leaving groups as substituents on the ring can be used to react first with the urease inhibitor complexes herein (instead of with dye). Such a reaction thereby forms materials which will then further react with cotton or wool fabrics during cleaning or treating operations. Examples of materials of this type are those used to form reactive dyes such as citrate-substituted triazine and other similar materials as disclosed in WO 99/51682 through WO 99/51689, all published October 14, 1999, and incorporated herein by reference.

The urease inhibitor-containing compositions as hereinbefore described may, when in liquid form, be provided for contact with objects, areas, substrates or environments to be treated therewith in the form of finely divided droplets

or particles. Thus liquid malodor control compositions herein may be packaged in or delivered via containers or devices which aerosolize or spray the composition in such particulate or droplet form as needed or desired.
Frequently such products in sprayable form will provide droplets which range in size from 1 to 5 microns. Sprayable compositions are suitable, for example, for application in droplet form to hard surfaces or fabrics.

Another suitable type of composition which can usefully employ the selected urease inhibitors described herein to control urine-malodor comprises pet litter. Pet litter, and in particular cat litter, will generally comprise an absorbent solid material as an essential component.
Suitable examples of such an absorbent materials include minerals, typically clays such as kaolinites, montmorillonites or bentonites; fly ash as obtained from the burning of coal; absorbent fibrous webs like cellulose webs or polymeric fibrous webs; pelletized absorbent materials (e.g., sawdust or polyurethane foam); and the like.
Particle sizes typically range from 0.25 cm to 1.3 cm.
Other examples of suitable solid absorbent materials are disclosed in U.S. 3,921,581, issued November 25, 1975 to Brewer, incorporated herein by reference.

Pet litter compositions may contain other components as well, including non-absorbent materials and odor control agents of a variety of types. Pet litter may also be employed in devices such as litter boxes of a wide variety of configurations. Such additional materials and litter box configurations can include, for example, those disclosed in U. S. Patents 5,031,578 and 4,517,919, incorporated herein by reference.

The urease inhibitor complexes used in the present invention may be added to conventional pet litter compositions in amounts which range from 0.1% to 10% by weight of the total pet litter composition. Such urease inhibitor complexes may be associated with any compositional or apparatus component of the pet litter composition or container therefor.

In another compositional aspect of the present invention, the selected urease inhibitor complexes described herein may be used to treat animal waste to provide compositions in the form of low odor fertilizer. In this context, the urease inhibitor complex can be sprayed onto animal waste or otherwise admixed therewith such that the urease inhibitor complex comprises from 0.1% to 10% by weight of the admixture. Fertilizer compositions such as these which are based on treated animal waste present less of an odor problem than untreated animal waste. Treatment with the urease inhibitor complexes herein has the additional advantage of stabilizing the treated fertilizer material with respect to its nitrogen content.

## Malodor-Controlling Articles

The selected urease inhibitor complexes described herein my also be utilized as urine or sweat malodor control agents in a wide variety of articles and devices. A number of specific types of such articles and devices are described as follows:

Perhaps the most common types of articles wherein urine-malodor control can be of special importance are wearable absorbent articles for a discharged body fluids. Such articles include infant diapers, adult incontinent devices and catamenial products. Generally absorbent articles of this type comprise an absorbent core positioned between a fluid impervious backsheet and a fluid pervious topsheet. The absorbent cores are generally fashioned from hydrophilic absorbent material such as cellulose fibers, e.g., wood pulp, and may contain materials such as gelling agents or absorbent foams serve to retain absorbent body fluids even under varying conditions of pressure and movement by the wearer of the article. Wearable articles for absorbing and holding discharged body fluids are described in greater detail in U.S. Patents 4,610,678; 4,657,537 and 4,842,593, all incorporated herein by reference.

In the context of absorbent articles for discharged body fluid such as diapers, incontinence devices and catamenial products, the selected urease inhibitor complexes disclosed herein may be incorporated into or onto such articles in any suitable way by any suitable means. Thus the urease inhibitor complexes may be associated with the topsheet, backsheet or absorbent core of such articles or may be associated with a separate additional element added to the article. The urease inhibitor complexes may be added to such articles in an aqueous solution or a non-aqueous (dry) form. The complexes may be absorbed into, adsorbed onto or brought into contact with one or a number of the elements of the absorbent article. As described more fully hereinafter, the urease inhibitor complexes used in this invention may also be chemically linked or bonded to one or more of the elements of such articles. Generally, the urease inhibitor complex will be associated with such absorbent articles to the extent of from 0.1 mg to 100 mg per gram of article, more preferably from 0.2 mg to 10 mg per gram of article.

The malodor-controlling urease inhibitor complexes disclosed herein may also be associated with a wide variety of other types of absorbent or non-absorbent articles which are likely to come into contact with excreted or secreted body fluids or residues thereof. Such articles may be wearable or non-wearable and can include such items as sweatbands, socks, underwear, bed sheets, mattress covers, pillow cases, hand and bath towels, underarm pads, surgical gowns and drapes, wiping cloths, baby wipes, carpets, brushes, mops, paper towels and the like. The amount of urease inhibitor complex associated with such articles can very widely depending upon the article configuration and

end use. Typically, however, effective malodor control can be realized when the article contains from 0.1% to 10% by weight of the total dry article of the urease inhibitor complex.

As with the malodor-controlling absorbent articles for discharged body fluids, the urease inhibitor complex can be associated with the foregoing articles in any manner which is suitable for providing the urease inhibitor complex in an amount and arrangement which is effective for inhibiting malodor. As described above with respect to urease inhibitor complexes which can be made substantive to certain substrates, an especially effective way of incorporating the complex into any of the foregoing articles which contain cellulosic or wool materials is via a covalent bonding or grafting mechanism. In addition to the mechanisms discussed hereinbefore involving silane bonds or bonding through heterocyclic nitrogen moieties, the urease complexes herein can be grafted directly to wool or cellulosics such as cotton or paper via other bonding reactions. For example, HEDTA inhibitors grafted to cotton through bis-epoxy materials such as bis-epoxide 1,4-butanediol diglycidyl ether would provide grafted substrates of the formula:

$$\text{Cotton-O-}CH_2\text{-}CH(OH)\text{-}CH_2\text{-O-}(CH_2)_4\text{-O-}CH_2\text{-}CH(OH)\text{-}CH_2\text{-}N(CH_2\text{-}COOH)\text{-}(CH_2)_2\text{-}N(CH_2COOH)_2.$$

Such a chemically modified substrate, upon chelation of metal ion, would provide a material which could be used to fashion anti-urine malodor garments or devices.

Examples

The odor-controlling methods, compositions and articles of the present invention are illustrated by the following examples:

**EXAMPLE I**

The ability of a preferred chelated metal complex of the present invention to prevent the enzymatic degradation of urea by urease is demonstrated by a procedure involving detection of ammonia which may be produced by this reaction. In such a test, conventional paper towel sheets are treated with both a urea solution and a urease solution. One such sheet serves as a reference sheet; another such sheet is treated with a urease solution which also contains a complex of copper chelated with N-(2-hydroxyethyl)ethylenediamine triacetic acid. After 30 minutes at room temperature, the sheets are evaluated for detectable ammonia order.

The reagents, solutions made therefrom and test sheets are prepared and tested as follows:

**Reagents**

- **Urea** : (Riedel-de Haen, extra pure, ref 16064, Cas#57-13-6)
- **Trizma Base** : (Amine-based buffer; Sigma, reagent grade, cat No T1503, CaB#77-86-1)
- **HEDTA** or N(2-Hydroxyethyl)ethylenediamine triacetic acid trisodium salt hydrated, 99%
  : (Aldrich Cat.No16.153-5, Cas#150-39-0
- **$CuCl_2$ dihydrate** : (99%, ACS reagent (Aldrich catno. 30,748-3, Cas#10125-13-0)
- **Urease** : (Type III from jack beans, Sigma ref U-1500)
- **Deionized water**

**Solutions**

- Tris buffer 0.2M, pH 7.5
- Dissolve 2.42g of trizma-base in about 80mL $H_2O$ deionized, adjust the pH to 7.5 with HCL 1M, bring to 100mL with $H_2O$ deionized.
- Urea 2M in Tris buffer
  Dissolve 6g of Urea in 50 mL of this buffer 0.2M, pH 7.5
- $CuCl_2$ solution (250mM)
  Dissolve 2.13g of CuCl in 50 mL of $H_2O$ deionized.
- HEDTA solution (250mM)
  Dissolve 2.15g of HEDTA in 25mL of $H_2O$ deionized

- Cu/HEDTA complex solution (125mM)
  Mix 25 ml of $CuCl_2$ solution (250mM) with 25 mL HEDTA solution (250mM) at adjusted with NaOH to pH around 7
- Urease solution (10mg/mL)
  Weigh 0.020g of urease and add 3mL of tris buffer pH 7.5, 0.2M. This solution is stable for about 1 week provided it is stored at 4°C in a refrigerator.
- Reference Solution
  10mL urea 2M/tris + 500mL of $H_2O$ deionized buffer.
- CuHEDTA Test Solution
  10mL Urea 2M/tris + 500mL Cu/TED complex solution buffer (125mM)

**Test Procedure**

Half of a white BOUNTY™® towel sheet is put in each of two Petri dishes. Into one dish, 15 drops of the reference solution are poured evenly so that the center of the towel sheet is evenly wet. The same procedure is used with the other dish except that 15 drops of the CuHEDTA Test Solution are used. Two drops of urease solution are then added to each dish, and the dishes are closed.

After 30 minutes at room temperature, a strong ammonia smell develops in the reference dish. No ammonia odor is detectable in the test solution dish.

**EXAMPLE II**

A liquid product for treating hard surfaces is prepared and tested by spraying the product on walls, floors and other surfaces surrounding the urinals in a public men's restroom. Product preparation, use and test results are as follows:

A 5mMol/liter solution of CuHEDTA is prepared from:

1721mg of HEDTA (trisodium salt hydrate, MW=344.21);
1250mg of $CuSO_4$ (pentahydrate, MW=250) ;
1000ml of deionized water.

Using a spray-gun, 250ml of this solution is sprayed once per day for 11 days over a $4m^2$ area surrounding the urinals in a public men's restroom. Assuming that the molecular weight of CuHEDTA is 339 and the concentration of the CuHEDTA solution is 5mMol/liter, then the amount of CuHEDTA applied is $105mg/m^2$. After such treatment, urine malodor in the men's restroom is effectively controlled for the full 11 day period of the test.

**EXAMPLE III**

A cotton swatch of fabric is grafted to a pentadentate chelant moiety and then tested for its ability to provide a urine malodor control benefit. Preparation and testing of the grafted cotton swatch are described as follows:

1. Cotton Fabric Activation Step

Cotton fabric is activated in alkaline medium by reaction with the bis-epoxide 1,4-butanediol diglycidyl ether according to the following reaction:

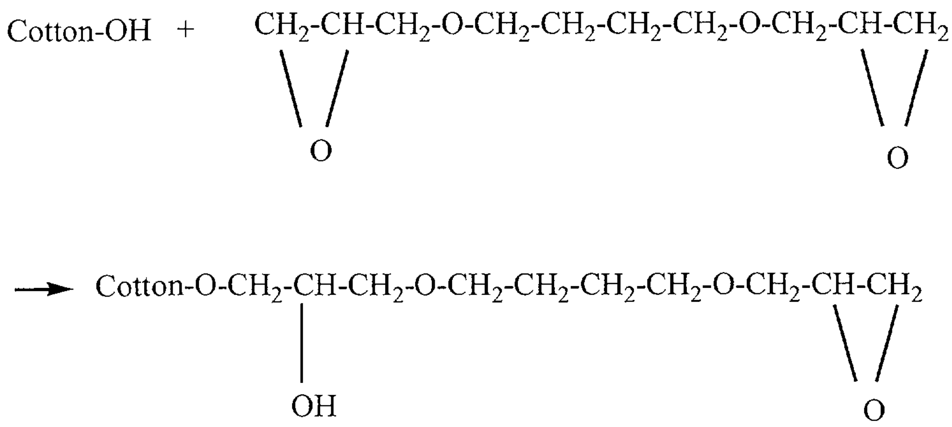

2. <u>Coupling Step</u>

For coupling, the remaining epoxide function is reacted under alkaline conditions with ethylenediamine (ED) according to the following reaction:

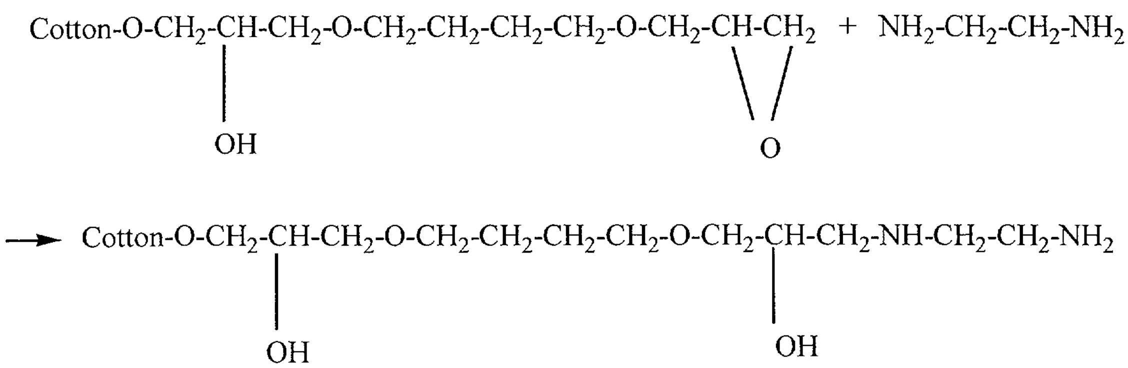

3. <u>Synthesis of the HEDTA Pentadentate Chelant</u>

The tris(carboxymethyl)ethylenediamine derivative is obtained by reacting the ED-grafted cotton with bromoacetic acid to yield:

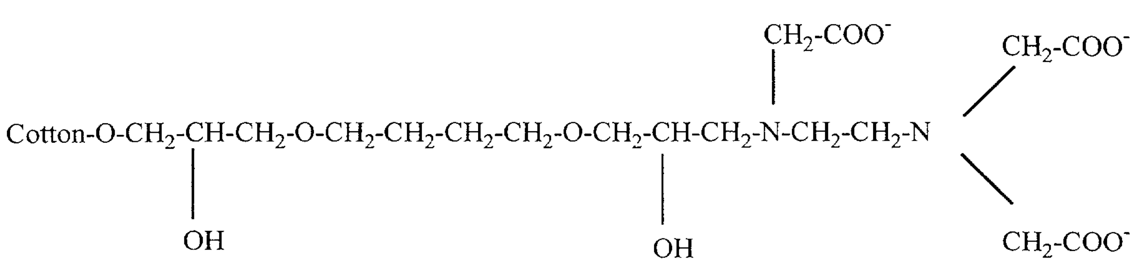

This HEDTA-derivatized cotton is then saturated with $Cu^{2+}$ ions by soaking the cotton by $CuCl_2$ solution and then rinsing the excess $CuCl_2$ with water. This procedure results in a HEDTA pentadentate chelant of the formula:

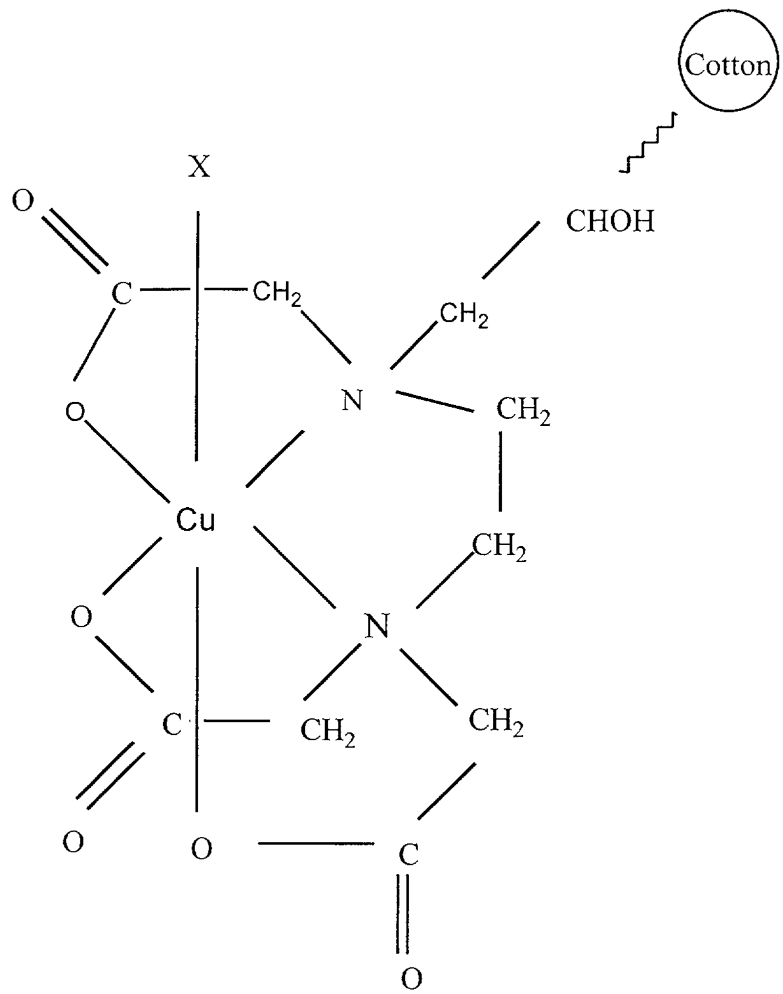

wherein X is a coordination site on the copper ion available for binding urease.

4. Simulated Urine Malodor Control Testing

The CuHEDTA-grafted cotton swatch is impregnated with a solution of urea (2M)and jack bean urease solution (See Example I). This swatch is then incubated at 40°C for 15 minutes. The release of ammonia resulting from urea degradation by urease is olfactory assessed and indicates almost no ammonia is released from the grafted cotton.

## Claims

1. A method for preventing or minimizing ammonia odor produced by degradation of urea in secreted or excreted body fluids, which method is **characterized in that** it comprises bringing into contact with urea-containing body fluids, or residues thereof, an effective amount of a urease inhibitor comprising a complex formed from a polyanionic chelating agent and a divalent metal ion wherein said metal ion is complexed at from 4 to 6 coordination sites with one additional coordination site remaining available for binding with urease and wherein said complex has a stability constant K such that log(10) K > 12.5.

2. A method according to Claim 1 wherein said chelating agent is an amine-based chelating agent and said contact is brought about by contacting substrates insulted with said body fluids or residues with a composition containing said urease inhibitor complex.

3. A method according to Claim 2 wherein said substrate comprises hard surfaces and said urease inhibitor complex is delivered to said hard surfaces via a composition in liquid form.

4. A method according to Claim 3 wherein said liquid composition is sprayed onto said high surfaces in the form of fine droplets.

5. A method according to Claim 1 wherein said chelating agent is an amine-based chelating agent and wherein said contact is brought about by bringing body fluids or residues thereof into contact with an article which has associated therewith said urease inhibitor complex.

6. A composition suitable for preventing or minimizing ammonia odor produced by degradation of urea in secreted or excreted body fluids, which composition is **characterized in that** it comprises:

A) an effective amount of a urease inhibitor comprising a complex formed from a polyanionic chelating agent and a divalent metal ion wherein said metal ion is complexed at from 4 to 6 coordination sites with one additional coordination site remaining available for binding with urease and wherein said complex has a stability constant K such that log(10) K > 12.5; and

B) an effective amount of an agent suitable for delivering said urease inhibitor complex into contact with urea-containing body fluids or residues thereof.

7. A composition according to Claim 6 wherein said chelating agent is an amine-based chelating agent and said delivery agent comprises a liquid carrier or a solid, preferably granular, carrier.

8. A composition according to Claim 6 or Claim 7 wherein said composition is in the form of an aqueous solution which contains from 0.1% to 10% by weight of said urease inhibitor complex.

9. A composition according to any of Claims 6 to 8 wherein said composition is in the form of a cleaning composition which contains from 0.1% to 50% by weight of a detersive surfactant and/or from 0.1% to 80% by weight of a detergent builder.

10. A composition according to any of Claims 6 to 9 wherein said urease inhibitor complex is chemically modified to render it substantive to a substrate upon contact of said substrate with said composition.

11. A composition according to Claim 10 wherein said urease inhibitor complex is chemically reactive with siliceous hard surfaces upon contact of said composition with said hard surfaces.

12. A composition according to Claim 10 wherein said urease inhibitor complex is chemically reactive with fabrics upon contact of such fabrics with said composition.

13. A composition according to Claim 12 wherein said urease inhibitor complex is rendered reactive with fabrics via a heterocyclic nitrogen moiety.

14. A composition according to Claim 6 or Claim 7 which is in the form of a pet litter composition which contains solid absorbent material and from 0.1% to 10% by weight of said urease inhibitor complex.

15. A composition according to Claim 6 or Claim 7 which is in the form of a stabilized animal waste-based fertilizer composition and which comprises urea-containing solid animal waste and from 0.1% to 10% by weight of said urease inhibitor complex.

16. An article suitable for preventing or minimizing ammonia odor produced by degradation of urea in secreted or excreted body fluids, which article is **characterized in that** it comprises:

A) an effective amount of a urease inhibitor comprising a complex formed from a polyanionic chelating agent and a divalent metal ion wherein said metal ion is complexed at from 4 to 6 coordination sites with one additional coordination site remaining available for binding with urease and wherein said complex has a stability constant K such that log(10) K > 12.5; and
B) means suitable for delivering said urease inhibitor complex into contact with urea-containing body fluids or residues thereof.

17. An article according to Claim 16 wherein said chelating agent is an amine-based chelating agent and wherein said

article is in the form of a wearable absorbent article for discharged body fluids, which wearable absorbent article comprises an absorbent core positioned between a fluid impervious backsheet and a fluid pervious topsheet.

**18.** An absorbent article according to Claim 17 wherein said urease inhibitor complex delivery means comprises having said urease inhibitor complex associated with said absorbent core, fluid impervious backsheet or fluid pervious topsheet and wherein said article comprises from 0.1 mg to 100 mg of said urease inhibitor complex per gram of said article.

**19.** An article according to Claim 16 wherein said chelating agent is an amine-based chelating agent; wherein said article is in the form of a sweatband, sock, underwear, bed sheet, mattress cover, pillow case, hand or bath towel, underarm pad, surgical gown or drape, wiping cloth, carpet, brush, mop or paper towel; and wherein said urease inhibitor complex comprises from 0.1% to 10% by weight of said article.

**20.** An article according to any of Claims 16 to 19 wherein said urease inhibitor complex is chemically bonded to one or more elements of said article.

**21.** An article according to Claim 20 wherein said urease inhibitor complex is covalently bonded to a cellulosic element of said article via reaction with a bis-epoxy compound.

**22.** A method, composition or article according to any of Claims 1 to 21 wherein said divalent metal ion is selected from copper, iron, zinc, cobalt or nickel.

**23.** A method, composition or article according to any of Claims 1 to 22 wherein said chelating agent is selected from nitrilotriacetic acid, iminodisuccinic acid, and substituted ethylenediamine materials of the general formula

$$R(CH_2COOH)N\text{-}(CH_2)_2\text{-}N\text{-}(CH_2\text{-}COOH)_2$$

wherein R is an organic moiety which does not form a coordination link with the heavy metal ion to be chelated therewith.

**24.** A method, composition or article according to any of Claims 1 to 23 wherein said urease inhibitor complex is copper N-(2-(hydroxyethyl)ethylenediamine triacetate.

**25.** A method for preventing or minimizing ammonia odor produced by degradation of urea in secreted or excreted body fluids, which method is **characterized in that** it comprises bringing into contact with urea-containing body fluids, or residues thereof, an effective amount of a urease inhibitor comprising a pentadentate chelant complex formed by chelating copper with N-hydroxyethyl-ethylenediamine-triacetic acid or iminodisuccinic acid.

**26.** A composition suitable for preventing or minimizing ammonia odor produced by degradation of urea in secreted or excreted body fluids, which composition is **characterized in that** it comprises:

A) an effective amount of a urease inhibitor comprising a pentadentate chelant complex formed by chelating copper with N-hydroxyethyl-ethylene-diamine-triacetic acid or iminodisuccinic acid; and

B) an effective amount of an agent suitable for delivering said urease inhibitor into contact with urea-containing body fluids or residues thereof.

**27.** An article suitable for preventing or minimizing ammonia odor produced by degradation of urea in secreted or excreted body fluids, which article is **characterized in that** it comprises:

A) an effective amount of a urease inhibitor comprising a pentadentate chelant complex formed by chelating copper with N-hydroxyethyl-ethylene-diamine-triacetic acid or iminodisuccinic acid; and

B) means suitable for delivering said urease inhibitor complex into contact with urea-containing body fluids or residues thereof.

European Patent Office

# EUROPEAN SEARCH REPORT

Application Number
EP 00 87 0301

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | US 5 547 676 A (NOEL HUGUES ET AL) 20 August 1996 (1996-08-20)<br>* column 1, line 31 - column 2, line 5 *<br>* claims * | 1-3, 5-10,16, 22-27 | A01K1/015<br>A61L9/01<br>A61L15/46 |
| X | EP 0 123 489 A (PROCTER & GAMBLE ;PROCTER & GAMBLE EUROP (BE)) 31 October 1984 (1984-10-31)<br>* page 4, last paragraph - page 5, line 16 *<br>* page 21, last paragraph *<br>* claims * | 1,2, 5-10,12, 23-26 | |
| X | WO 99 45973 A (OSBORNE SCOTT EDWARD ;PROCTER & GAMBLE (US); ROE DONALD CARROLL (U) 16 September 1999 (1999-09-16)<br>* page 9, line 13 - line 32 * | 1,2,5-8, 10-12, 14, 16-18, 22,23 | |
| X | DE 36 42 564 A (LION CORP) 9 July 1987 (1987-07-09)<br>* page 4, line 59 - line 64 *<br>* page 5, line 33 - line 37 *<br>* claim 1 * | 1,2, 5-10,12, 22,23 | TECHNICAL FIELDS SEARCHED (Int.Cl.7)<br>A01K<br>A61L |
| X | WO 98 27261 A (MOWRY LESLIE ANDERSON ;TORDIL HELEN BERNARDO (US); LIU ZAIYOU (US)) 25 June 1998 (1998-06-25)<br>* page 6, line 12 - page 12, last line *<br>* page 39, line 21 - line 27 *<br>* claims 1-3,9 * | 1,2,5-7, 9,10, 14-16, 22,23, 26,27 | |

The present search report has been drawn up for all claims

| Place of search | Date of completion of the search | Examiner |
|---|---|---|
| THE HAGUE | 5 June 2001 | Thornton, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 00 87 0301

This annex lists the patent family membersrelating to the patent documents cited in the above-mentioned European search report.
The members are as contained in the European Patent Office EDP file on
The European Patent Office is in no way liable for these particulars which are merely given for the purpose of information.

05-06-2001

| Patent document cited in search report | Publication date | Patent family member(s) | Publication date |
|---|---|---|---|
| US 5547676 A | 20-08-1996 | FR 2697161 A | 29-04-1994 |
| | | CN 1090168 A,B | 03-08-1994 |
| | | DE 69307267 D | 20-02-1997 |
| | | DE 69307267 T | 28-05-1997 |
| | | EP 0595690 A | 04-05-1994 |
| | | ES 2096248 T | 01-03-1997 |
| | | GR 3023067 T | 30-07-1997 |
| | | JP 6211641 A | 02-08-1994 |
| | | KR 196310 B | 15-06-1999 |
| EP 0123489 A | 31-10-1984 | AT 42112 T | 15-04-1989 |
| | | CA 1224996 A | 04-08-1987 |
| | | DE 3477675 D | 18-05-1989 |
| | | ES 531735 D | 01-12-1985 |
| | | ES 8602927 A | 16-03-1986 |
| | | GR 79856 A | 31-10-1984 |
| | | IE 57223 B | 03-06-1992 |
| | | JP 1815758 C | 18-01-1994 |
| | | JP 5018880 B | 15-03-1993 |
| | | JP 60035100 A | 22-02-1985 |
| | | US 4680131 A | 14-07-1987 |
| WO 9945973 A | 16-09-1999 | AU 3079599 A | 27-09-1999 |
| | | BR 9908565 A | 12-12-2000 |
| | | EP 1061962 A | 27-12-2000 |
| | | TR 200002601 T | 21-12-2000 |
| DE 3642564 A | 09-07-1987 | JP 2008536 C | 11-01-1996 |
| | | JP 6084515 B | 26-10-1994 |
| | | JP 62138597 A | 22-06-1987 |
| | | JP 62146999 A | 30-06-1987 |
| WO 9827261 A | 25-06-1998 | BR 9714042 A | 24-10-2000 |
| | | EP 0946808 A | 06-10-1999 |

EPO FORM P0459

For more details about this annex : see Official Journal of the European Patent Office, No. 12/82